# EUROPEAN PATENT APPLICATION

(11) **EP 2 537 506 A1**
(43) Date of publication of application: **26.12.2012**
(21) Application number: 12185031.7
(22) Date of filing: 15.10.2008
(51) Int. Cl.: A61J 3/07

(54) **Linkers for multipart dosage forms for release of one or more parmaceutical compositions, and the resulting dosage forms**

(30) Priority: 15.10.2007 US 960786 P
(62) Divisional of application: 08839618.9
(71) Applicant: Capsugel Belgium NV, 2880 Bornem (BE)
(72) Inventor: McAllister, Stephen Mark, Essex, CM 19 5AW (GB)
(74) Representative: Krishnan, Sri

(57) **Abstract**

A dosage form (100) comprising a first generally cylindrical capsule compartment (102) having an axis (104), a closed end (106), and an open end (108); the open end (108) including an integral, annular linker member (110) extending axially outward of the open end (108) and having an outer surface (120) with a first snap-fit element (122) located a distance L1 from the capsule compartment open end (108); a second generally cylindrical capsule compartment (120) having an axis (104), a closed end (123), and an open end (124) with an inner surface (130), the second capsule compartment (120) having a second snap-fit element (128) complementary to the first snap-fit element (122), the second snap-fit element (128) being located on the inner surface (130) and spaced a distance of L2 from the second capsule open end (124), wherein L2 > L1 and the first and second snap-fit elements (122, 128) are engageable to connect the first and second capsule compartments (102, 120) with the respective open ends (108, 124) being in compressive abutting contact with each other.

## Description

### FIELD OF THE INVENTION

This invention relates to pharmaceutical dosage forms and, more particularly, to multipart capsules including a linker unit and one or more connected sub- units for oral dosing.

### BACKGROUND OF THE INVENTION

Various types of pharmaceutical dosage forms are known for oral dosing. Such capsules generally comprise an envelope wall of a pharmaceutically acceptable, e.g. orally ingestible, polymer material such as gelatin, although other materials for capsule walls, e.g. starch and cellulose based polymers are also known. Such capsules generally have soft walls made by forming a film on a capsule former, which is then allowed to dry. Rigid walled capsules made by injection molding are also known; see for example U.S. 4,576,284, U.S. 4,591 ,475, U.S. 4,655,840, U.S. 4,738,724, U.S. 4,738,817, and U.S. 4,790,881 (all to Warner Lambert). These disclose specific constructions of capsules made of gelatin, starch and other polymers, and methods of making them by injection molding of hydrophilic polymer, e.g., water mixtures. U.S. 4,576,284 specifically discloses such capsules provided with a cap which closes the capsule and is formed in situ on the filled capsule by molding. U.S. 4,738,724 discloses a wide range of rigid capsule shapes and parts.

Multi-compartment capsules, including those of the type where each compartment has different drug release characteristics or, for example, contains a different drug substance or formulation, are also known; see for example U.S. 4,738,724 (Warner-Lambert), U.S. 5,672,359 (University of Kentucky), U.S. 5,443,461 (Alza Corp.), WO 9516438 (Cortecs Ltd.), WO 9012567 (Helminthology Inst. ), DE-A- 3727894, BE 900950 (Warner Lambert), FR 2524311 , NL 7610038 (Tapanhony NV), FR 28646 (Pluripharm), and U.S. 3,228,789 (Glassman), U.S. 3,186,910 (Glassman), among others. U.S. 4,738,817, U.S. 3,228,789, and U.S. 3,186,910 each disclose a multicompartment capsule made of a water-plasticized gelatin.

Pharmaceutical dosage forms that comprise a matrix of a solid polymer, in which a drug substance is dispersed, embedded or dissolved as a solid solution are also known. Such matrixes may be formed by an injection molding process. This technology is discussed in Cuff G. and Raouf F., Pharmaceutical Technology, June 1998, p. 96-106. Some specific formulations for such dosage forms are, for example disclosed in U.S. 4,678,516; U.S. 4,806,337; U.S. 4,764,378; U.S. 5,004,601 ; U.S. 5,135,752; U.S. 5,244,668; U.S. 5,139,790; U.S. 5,082,655 among others, in which a polyethylene glycol ("PEG") matrix is used and solid dosage forms are made by injection molding.

See, also for example, WO 01/08666, WO 02/060385, US 2004/0115256, US 2006/0049311 , WO 02/060384, US 2003/0068369, US 2004/0166153, WO 04/010978, US 2006/0057201 , WO 05/009380, US 2005/0175687, WO 05/089726, US 2005/0249807, US 60/968,383, US 61/061 ,275, and the content of PCT/EPOO/07295 entitled "MULTICOMPONENT PHARMACEUTICAL DOSAGE FORM". WO 01/08666 relates to multicomponent pharmaceutical dosage form comprising two or more connected sub-units, particularly for oral dosing.

### SUMMARY OF THE INVENTION

The present invention relates to a dosage form includes a first generally cylindrical capsule compartment having an axis, a closed end, and an open end, the open end including an integral, annular linker member extending axially outward of the open end and having an outer surface with a first snap-fit element located a distance L₁ from the capsule compartment open end. The dosage form also includes a second generally cylindrical capsule compartment having an axis, a closed end, and an open end with an inner surface, the second capsule compartment having a second snap-fit element complementary to the first snap-fit element, the second snap-fit element being located on the inner surface and spaced a distance of L₂ from the second capsule open end. L₂ is greater than L₁, and the first and second snap-fit elements are engageable to connect the first and second capsule compartments with the respective open ends in compressive abutting contact with each other.

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### BRIEF DESCRIPTION OF THE DRAWINGS

The invention will now be described by way of example with reference to:

Fig. 1 which is a perspective view of a linker made in accordance with one aspect of the present invention;

Fig. 2 is a cross section of a preformed tablet component of the linker depicted in Fig. 1 ;

Fig. 3 shows various perspective views of the preformed tablet component shown in Fig. 2;

Fig. 4 is a cross sectional view of the linker shown in Fig. 1 and having a closing wall at one end;

Fig. 5 shows various perspective views of the linker shown in Fig. 4;

Fig. 6 is an exploded view of a multipart dosage form utilizing the linker shown in Figs. 1 and 2;

Fig. 7 is a cross sectional view of an assembled dosage form including the linker shown in Fig. 1 ;

Fig. 8 is an enlarged view of detail A in Fig. 7;

Fig. 9 shows an alternative construction of the groove-type snap-fit element shown in Fig. 8;

Fig. 10 is an exploded view of another embodiment of the present invention;

Fig. 11 shows various perspective and end views of the dosage form of Fig. 10 in an assembled state;

Fig. 12 depicts an exploded cross sectional view of the dosage form shown in Fig. 10;

### DETAILED DESCRIPTION

Reference will now be made in detail to the present embodiments of the invention, examples of which are illustrated in the accompanying drawings.

In accordance with one aspect of the present invention, a linker is disclosed for connecting with one or more dosage form units from the group including capsule compartments and closure caps, with the linker holding a drug substance. Specifically, the linker includes a solid drug substance in tablet form, the tablet having a longitudinal axis and being substantially cylindrical with opposed axial end faces. The linker further includes a jacket formed around and radially confining the tablet, the jacket having an outer wall with longitudinal ends, one or both of the jacket ends being opened for dispensing the drug substance from the respective end face. The linker still further includes the jacket outer wall a having snap-fit element adjacent at least one jacket longitudinal end, the snap-fit element preferably being selected from circumferential grooves, groove segments, ridges, and ridge segments.

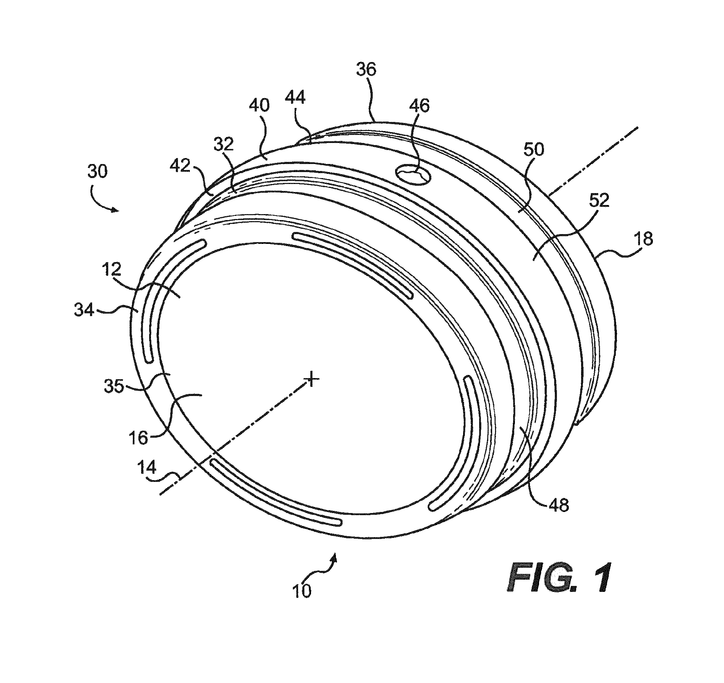

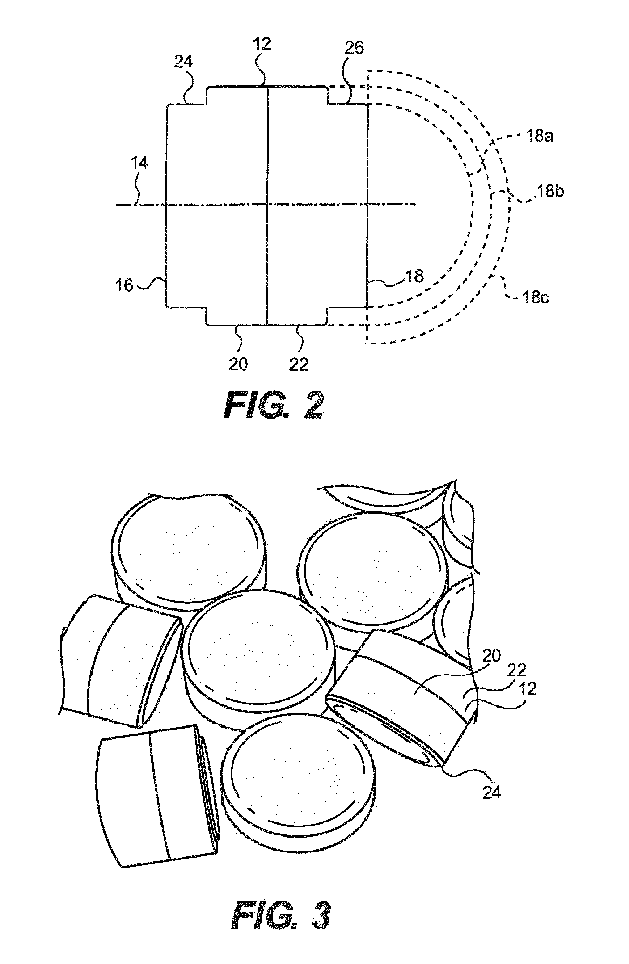

As embodied herein and with initial reference to Figs. 1-4, linker 10 includes tablet 12, which may be substantially cylindrical, having an axis 14 and closed axial end faces 16 and 18. End faces 16 and 18 may be generally planar and perpendicular to axis 14, or one or both may be shaped to extend axially, for reasons to be discussed below, such as rounded end face 18a, 18b, or 18c depicted by dotted lines in Fig. 2. Tablet 12 preferably is preformed outside linker 10 by processes such as dry compacting, casting, or other process known in the art. Tablet 12 can be composed of a single drug substance or, as shown in Fig. 2, a bi-layer configuration can be used having drug substance parts 20, 22. The drug substances in parts 20 and 22 may differ in composition and/or release characteristics, and can be appropriately indicated as such by coloration to ensure correct assembly into dosage forms, as will be discussed below. Also, it is preferred that tablet 12 includes recessed lands 24, 26 formed around respective perimeters of tablet end faces 16, 18, for securing jacket 30 of linker 10 as will be discussed below.

Drug substances for use in dosage forms suitable for being administered orally to a patient can be included in a linker 10 and, in particular, that can be used in parts 20 and 22, can include any suitable or conventional form, such as, for example, a powder, granules, compact, microcapsules, gel, syrup, or liquid, provided that the capsule portion wall material is sufficiently inert to the liquid content of the latter three forms.

As embodied herein and with continued reference to Figs. 1 , 4 and 5, linker 10 includes a jacket 30 with a generally cylindrical outer wall 32 and respective longitudinal ends 34, 36. Jacket 30 may be constructed by injection molding around tablet 12 in order to leave one or both jacket ends 34, 36 open and to expose tablet end face 16 and/or 18 for dispersion and dissolution of the contained drug substance(s) once a connected capsule and/or cap dosage form unit has been breached, such as, for example, by changing shape, form, or structure within a gastro-intestinal environment, e.g., dispersing, dissolving, disintegrating, swelling, being partially or completely soluble, or otherwise changeable when exposed to stomach pH and/or in intestine pH. It is contemplated that jacket 30 may be injection molded around tablet 12 and that tablet 12 may be supported within a mold by, for example, one or more finger or gripper elements extending outward from a mold wall into the interior of the mold and engaging a portion of tablet 12, e.g., engaging the radially outer wall of tablet 12.

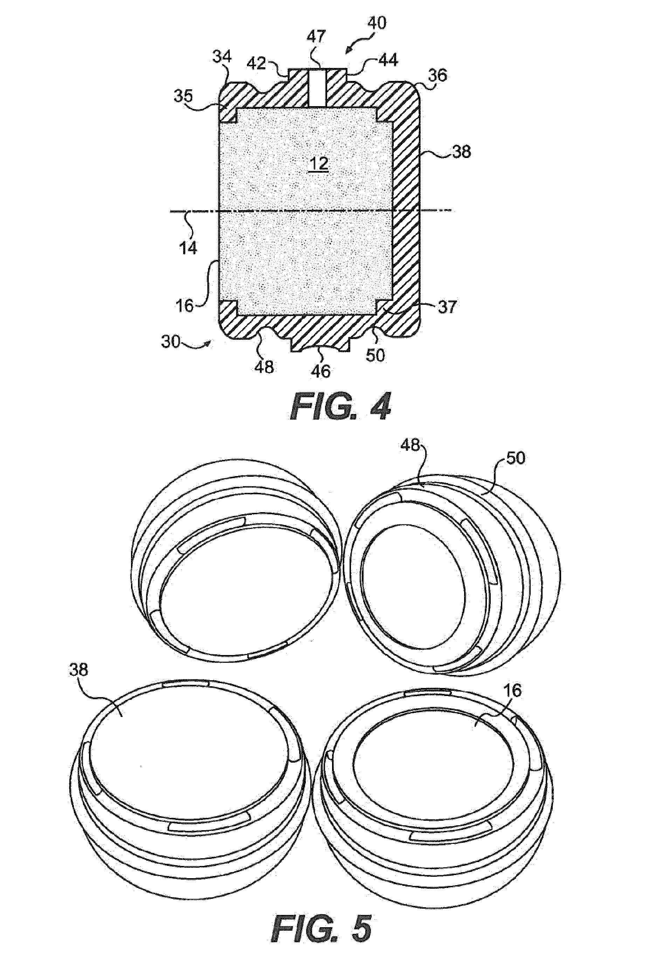

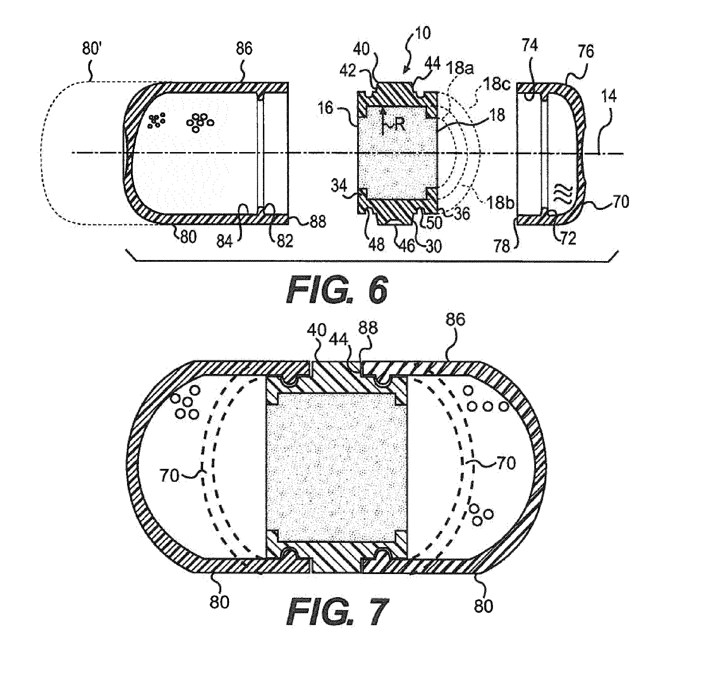

For example, in linker 10, shown in cross-section in Fig. 6, both jacket ends 34, 36 are open to expose tablet end faces 16, 18 for drug substance dissolution and/or dispersion, when cap unit 70 and/or capsule unit 80 are breached. In comparison, Figs. 4 and 5 show a variation in jacket 30 with a solid wall 38 closing off jacket end 36, to substantially prevent the drug substance in tablet 12 from dissolving/dispersing therethrough. Wall 38 can be integrally formed with the remainder of jacket 30 via injection molding. In both variations the injection molded jacket material radially confines a tablet 12 (radial direction being depicted in Fig. 6 as indicated by arrow R) and also axially confines tablet 12 to at least some degree as a result of jacket material flowing into the recess lands 24, 26 of tablet 12 and forming circumferential jacket end edges 35, 37. It is contemplated that a radially confining injection molded jacket includes the jacket surrounding at least a portion and contacting at least a portion of the circumference of the tablet, and may, for example, include the jacket being injection molded around the tablet or other method providing a jacket radially confining the tablet. It is also contemplated that slots 90 may be formed in jacket 30 at one or both of jacket ends 34, 36 due to tablet 12 being supported within a mold by the fingers or grippers (discussed above) during injection molding and which may aid in dissolution/dispersion of the tablet. See Figs. 1 and 5. Although four slots are illustrated in jacket end 34 (see Fig. 1 ), it is contemplated that any number of slots may be formed. [044] As further embodied herein, and referring again to Fig. 1 , jacket 30 includes a raised band 40 circumferential Iy formed on the periphery of outer jacket wall 32, preferably midway between longitudinal ends 34 and 36. Band 40 includes opposed side surfaces 42, 44 configured for abutting contact with the wall ends of dosage form units, e.g., cap unit 70 and/or capsule unit 80, interconnected with linker 10. As depicted in Figs. 1 and 4-6, side surfaces 42, 44 may be essentially perpendicular to axis 14 and raised band 40 also can have one or more concave depressions 46 to accommodate an injection molding feedgate. Also, as shown in Fig. 4, raised band 40 may have one or more radially directed apertures 47 to provide a direct path for controlled, relatively early dispersion of the drug substance in tablet 12, prior to breach or dissolution of any cap or capsule covering exposed tablet end faces, such as end face 16 in Fig. 4. Apertures 47 may be sealed with a rapidly dissolving thin film or coating (not shown) to prevent contamination of the drug substance of tablet 12. Such a film or coating may be made from any Phama-acceptable polymer suitable for film coating.

As further embodied herein, jacket 30 includes snap-fit elements 48, 50 formed on outer surface 52 of jacket wall 32 between raised band 40 and respective jacket longitudinals ends 34, 36. As shown in Figs. 1 and 4, both snap-fit elements 48, 50 are circumferential grooves configured and dimensioned to engage complementary circumferential ridge or "bead" elements on the inner surfaces of the respective cap unit or capsule unit. For example, cap unit 70 shown in Fig. 6 has a continuous circumferential ridge or bead 72 formed on inner surface 74 of outer wall 76 adjacent wall end 78. Ridge 72 is configured to engage groove 50 in linker 10 by a "snap-fit inter-connection." By snap-fit inter-connection, it is meant that the resiliency of cap unit 70 expands to allow ridge 72 to pass over a jacket surface 52 and subsequently contracts to allow ridge 72 to engage or "snap" into groove 50.

Similarly, and as shown in Fig. 6, capsule unit 80 has a circumferential ridge 82 on inner surface 84 of capsule unit wall 86 adjacent wall end 88 configured and dimensioned to be complementary to groove 48 of linker 10 provide a snap-fit inter connection.

Referring to Figs. 6 and 7, it can be seen that linker 10 may make possible multiple dosage form configurations interconnected with a snap-fit connections, including two capsule units 80 joined by linker 10 (see Fig. 7); two cap units 70 joined by linker 10 (see Fig. 7 shown as dashed); or a cap unit 70 joined by linker 10 to a capsule unit 80 (see Fig. 6). However, linker 10 can be used with only a single joined capsule, such as capsule 80 in the embodiment depicted in Fig. 6 but without cap 70, such as if the linker 30 is configured to have a solid end wall, such as end wall 38 in the Fig. 4 embodiment. Moreover, if dissolution/dispersion of an increased quantity of the solid drug in linker 30 is desired, the dosage form in Fig. 6 may be used without end cap 70 with the end face 18 drug material extended axially past linker end. In such a variation, the tablet end face 18 also may be shaped to provide easier swallowing such as with a rounded, extended face such as 18a depicted with dashed line in Fig. 6. Other configurations of an extended tablet end face that may be desirable include those depicted in Fig. 6 as 18b and 18c. Extended end face 18b may be achieved by omitting recess 26 in tablet 12 (see Fig. 2) and jacket end edge 37 (see Fig. 4) and tapering the jacket and edge 36 to provide a smoother dosage form end. A "mushroom" shaped extended end face configuration in 18c is an alternative way to achieve a smooth dosage form end. Also, "exposed" tablet end face 18a, 18b, or 18c may be covered with a thin rapidly dissolving film or coating (not shown) to prevent contamination of the drug substance of tablet 12. Such a film or coating may be made from any Phama-acceptable polymer suitable for film coating.

Further, in each of the above-discussed variants where linker end 36 is to be left uncapped, a snap-fit connection adjacent linker end 36 such as groove 50 may not be needed and band 40 tapered axially, thereby possibly simplifying the construction and manufacture of the linker. However, if linker 30 is constructed without a jacket end edge, such as for extended tablet end faces 18b and 18c, then it may be desirable to use a film covering (not shown) at least over the extended tablet face and adjacent linker end 36 to help axially constrain tablet 18.

Fig. 6 also depicts the use of capsules of varying length, e.g., a longer length capsule 80' shown as dashed, making possible asymmetric dosage forms. Moreover, while Figs. 1 and 4-7 show substantially symmetrical linkers having outer wall diameters at longitudinal ends 34 and 36 substantially equal and configured for attaching capsule and/or cap units of essentially the same diameter, variations in linker 10 having different jacket wall diameters at ends 34 and 36 are contemplated to interconnect capsule units and/or cap units of different diameters, thereby making possible a dosage form asymmetric in width. See also discussion of linker 212" shown in Fig. 16 described below.

Moreover, it is contemplated that the dimensions of the capsules, end caps, and linkers along with the respective snap-fit elements, e.g., cap unit 70 and linker 30, including respective ridge 72 and groove 50 in Fig. 6, may be adjusted to achieve any degree of snap-fit and that the snap-fit is preferably a relatively permanent, locking interconnection therebetween. That is, although the parts could be separated with sufficient force, such separation is not intended in the normal course of utilization of the dosage forms by a consumer. It is also contemplated that the position of the groove and ridge elements could be reversed, and that complementary ridge segments and groove segments could be used instead of circumferentially continuous members. Also, although ridge segments could be used with continuous grooves, leakage of the contained drug substances might possibly occur. Further, although the complementary grooves and ridges/"beads" shown in Figs. 1 and 4-7 are generally rounded or circular in axial cross section, other shapes such as, for example, wedges, trapezoids, triangles, may be used.

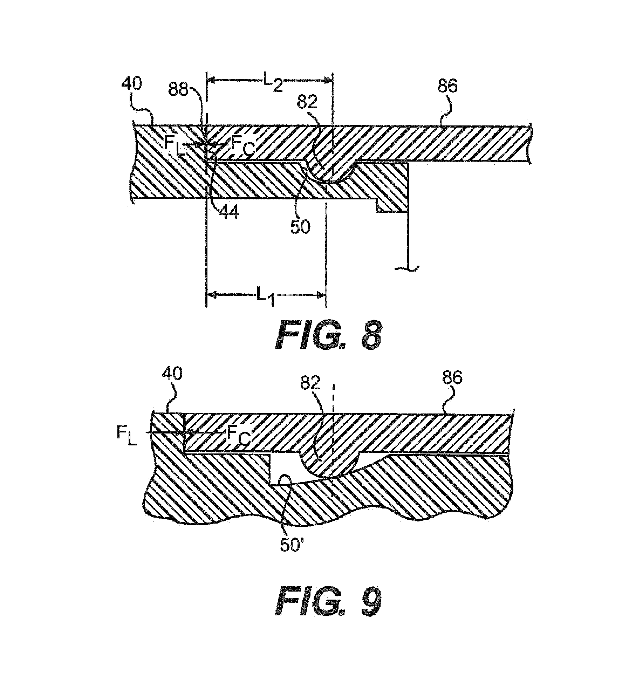

It is preferred that the linker, capsule units, and cap units be configured and dimensioned along with the snap-fit elements to provide a compressively abutting contact between the linker band 40, particularly band side surfaces 42, 44, and wall ends 78 and 88 of capsule unit 70 and cap unit 80, respectively. As best seen in Fig. 8, which is an enlarged view of detail A of Fig. 7, the distance L₁ between ridge 82 and the capsule wall end 88 may be slightly larger than the distance L₂ between groove 50 and band side 44 of linker 10. As such, upon engagement between the capsule wall end 88 and band side 44, the ridge 82 is not "fully seated" in groove 50. But as a consequence of the resiliency of cap end 86 an axial force tending to fully seat ridge 82 results in a force F_{c} exerted by a capsule wall and 88 against a linker band side 44 and a similar force F_{L} opposing this force. This compressively abutting cap contact may provide increased dosage form integrity, because it may eliminate any gap between the raised band 40 and the capsule end wall 88 which might otherwise establish a groove, edge, or other surface inconsistency that might increase a tendency to disengage the capsule unit from the linker. Also, the compressive abutting contact may minimize the chance for radial movement between the capsule unit and the linker and provide a relatively smooth surface to be established across the contact therebetween, potentially facilitating easier swallowing.

Still further, the compressive abutting contact provided by band 40 might provide a further barrier as a mechanical seal against unwanted drug substance leakage from the capsule compartments or the linker. This would be in addition to the seal provided by the contact between the snap-fit elements on the linker and the capsule compartments and/or closure caps. Also, the components of the multicompartment dosage forms can be configured to have a slight interference fit between the outer surface of the linker component and the inner surfaces of the capsule and/or cap units, to provide yet another barrier against leakage, while still providing ease of assembly.

Other means for providing a compressive abutting contact between the capsule and cap units against the linker band are contemplated including the use of different cross-sectional geometries of the grooves and/or ridges, as stated previously. Such a variation is shown in Fig. 9 where a wedge-shaped groove 50' is used instead of a circular cross-section. Again, it is contemplated that the one skilled in the art given this configuration and dimensions the linkers, capsules and/or cap units and associated snap-fit elements may be adjusted to achieve the desired compressive abutting contact function.

In accordance with another aspect of the present invention, a dosage form incorporating a linker component includes a first generally cylindrical capsule compartment having an axis, a closed end, and an open end. The open end includes an integral, annular linker member extending axially outward of the open end. The linker member has an outer surface with a first snap-fit element located a distance L₁ from the capsule compartment open end. The dosage form further includes a second generally cylindrical capsule compartment having an axis, a closed end, and an open end with an inner surface. A second snap-fit element complementary to the first snap-fit element is provided on the inner surface and spaced a distance of L₂ from the second capsule open end. L₂ is configured to be greater than L₁, and the first and second snap- fit elements are engageable to connect the first and second capsule compartments with respective open ends being in compressive abutting contact with each other.

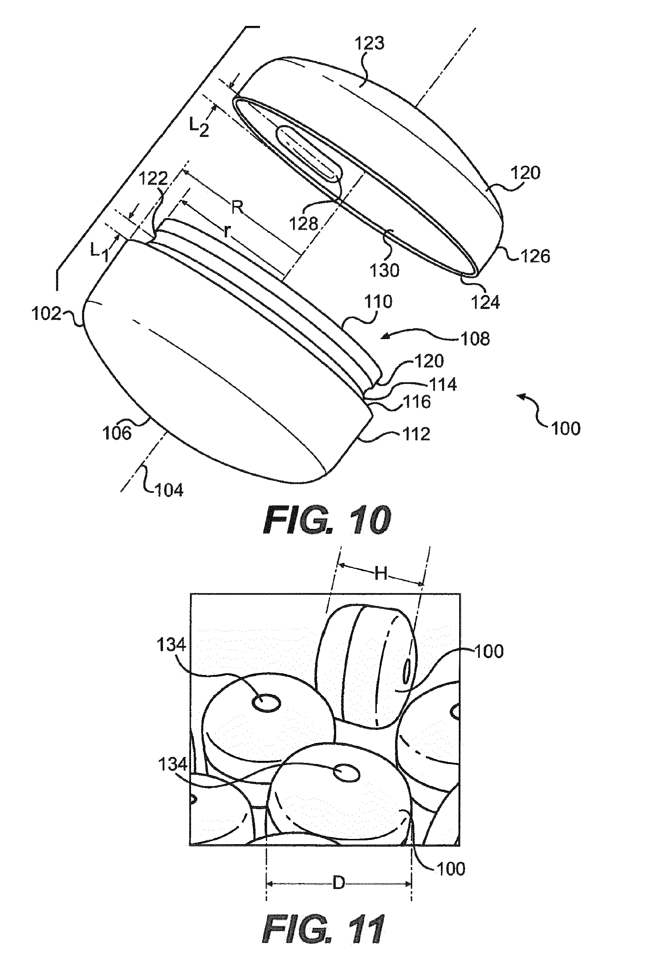

As embodied herein, Figures 10-12 illustrate a dosage form, designated generally as 100, that includes a first generally cylindrical capsule compartment 102 having axis 104. First capsule compartment 102 includes closed end 106 and open end 108. First capsule compartment 102 further includes annular linker member 110 fixed to an inner part 114 of the capsule compartment wall 112 and extending in the axial direction beyond outer wall 116, in a direction away from closed end 106. Linker member 110 is integral with first capsule compartment 102, such as, for example, by being consanguineous, by being injection molded together, being fused or welded, and/or connected via any other suitable method for integrally forming linker member 110 and first capsule compartment 102. Integral, as used herein, is intended to include substantially permanent connections, e.g., welds, between two elements and is not intended to include releasable connections, e.g., snap-fit connections, between two elements.

Linker member 110 further includes an outer surface 120 having a radius "r" less than the radius "R" of outer wall 112, as illustrated in Fig. 10. Circumferential groove 122 is formed on surface 120 at a distance L₁ from the end of outer wall 116, to act as a snap-fit element.

Dosage form 100 further includes a second capsule compartment 120 which is also generally cylindrical about axis 104 with closed end 123, open end 124, and outer wall 126. Three ridge segments 128 (only one being shown in Fig. 10) are formed on inner surface 130 of wall 126 circumferentially spaced about axis 104, to function as a complementary snap-fit element for engaging groove 122 of first capsule compartment 102.

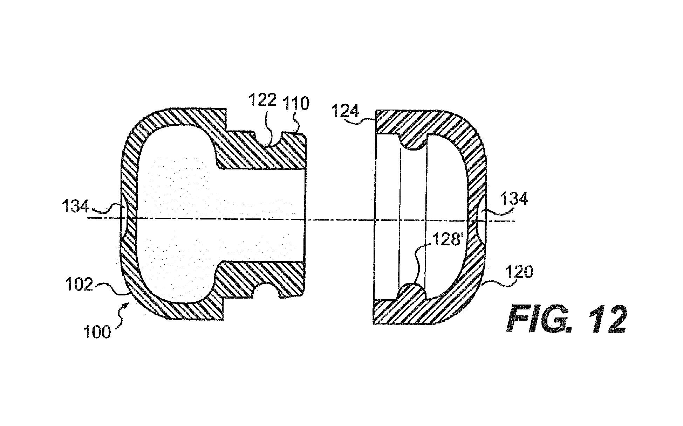

The dosage form 100 shown in Fig. 11 is configured as a tablet and has a height/diameter ratio less than or equal to 1.0. In order to ensure a continuous overall dosage form outer surface, the capsule compartments and linker member, including the respective snap-fit elements (groove 122 and ridge segments 128) are configured and dimensioned to achieve compressive abutting contact between outer wall part 116 of compartment 102 and outer wall 126 at open end 124 of compartment 120. The compressively abutting contact may be accomplished by specifying L₁ < L₂, as discussed previously in relation to the embodiment in Figs. 7 and 8. Also, as in the previously discussed embodiment, the positions of the complementary groove and ridge segment snap-fit elements can be interchanged. Moreover, a continuous ridge, such as ridge 128' (as shown in Fig. 12) can be substituted for ridge segments 128 for use with the continuous groove 122. Or, groove segments (not shown) could be substituted for use with ridge segments 128, as previously discussed.

First capsule compartment 102, including linker element 110, and second capsule compartment 120, together with their respective snap-fit elements, can be formed by injection molding. Also, depressions in the walls of compartments 102 and 120 can be provided to accommodate injection material overflow and retain a smooth overall dosage form surface, such as depressions 134 at the closed compartment ends (see Fig. 11 ).

Each of the capsule compartments, closure caps, linkers, and linker parts may be made of a transitional polymer and may comprise the same or different polymer. A transitional polymer is a polymer that changes shape, form, or structure within a gastro-intestinal environment, e.g., dispersible, dissolvable, disintegrable, breachable, swellable, partially or completely soluble, fracturable, or otherwise changeable when exposed to stomach pH and/or in intestine pH. Suitable polymers include: polyvinyl alcohol (PVA), natural polymers (such as polysaccharides like pullulan, carrageenan, xanthan, chitosan or agar gums), polyethylene glycols (PEG), polyethylene oxides (PEO), mixtures of PEGS and PEOS, hydroxypropylmethylcellulose (HPMC), methylcellulose, hydroxyethylcellulose, hydroxyethyl methylcellulose, hydroxypropylcellulose, methacrylic acid copolymer (such as Eudragit E^{™}, Eudragit L^{™} and/or Eudragit S ^{™}), ammonium methacrylate copolymers (such as Eudragit RL^{™} and/or Eudragit RS ^{™}), carboxymethylcellulose, povidone (polyvinyl pyrrolidone), polyglycolysed glycerides (such as Gelucire 44/14^{™}, Gelucire 50/02^{™}, Gelucire 50/13^{™} and Gelucire 53/10^{™}), carboxyvinyl polymers (such as Carbopols^{™}), polyoxyethylene- polyoxypropylene copolymers (such as Poloxamer 188^{™}), and acrylic and/or methacrylic acid-based polymers. The Eudragit^{™} polymers discussed above for example are extrudable and may for example be plasticised with e.g. triethyl citrate, or glyceryl monostearate.

Preferred polymers are orally ingestible polymers and include hydroxypropyl methylcellulose acetate succinate (HPMC-AS), polyvinyl alcohol, hydroxypropyl methyl cellulose, and other cellulose-based polymers. Preferred polymers also include polymer materials which preferentially dissolve or disintegrate at different points in the digestive tract. Such polymers include the known acrylic and/or methacrylic acid-based polymers which are transitional in intestinal fluids, e.g. the Eudragit series of commercially available polymers. Examples of these include Eudragit E^{™}, such as Eudragit E 100 ^{™} or Eudragit 4135F^{™}, which preferentially dissolves in the more acid pH of the stomach, or enteric polymers such as Eudragit L ^{™} and/or Eudragit S^{™} which preferentially dissolve in the more alkaline pH of the intestine, and preferred polymers also include polymers which dissolve slowly, e.g. at a predetermined rate in the digestive tract, such as Eudragit RL^{™} e.g. Eudragit RL 100 ^{™}, and/or Eudragit RS ^{™} e.g. Eudragit R100 ^{™}, and/or blends of such Eudragit ^{™} polymers.

The polymers may include other substances to modify their properties and to adapt them to various applications, including, for example, the following general classes of substances: surfactants, such as Polysorbate 80^{™}, sodium lauryl sulphate, and Polyoxyl 40 ^{™} hydrogenated castor oil; absorption enhancers, such as Labrasol ^{™}, Transcutol^{™}; glidants, such as stearyl alcohol, talc, magnesium stearate, silicon dioxide, amorphous silicic acid, fumed silica, Simeticone^{™}; plasticizers, such as triethyl citrate, acetyl triethyl citrate, tributyl citrate, acetyl tributyl citrate, glyceryl monostearate, diethyl phthalate, dibutyl phthalate, propylene glycol, triacetin and castor oil; substances for release modification, such as ethyl cellulose and cellulose acetate phthalate; disintegrants, such as sodium starch glycollate, croscarmellose sodium, crospovidone (cross-linked polyvinyl pyrrolodone), coloring agents, flavoring agents and sweetening agents.

It is contemplated that in addition and/or as an alternative to the full or partial circumferential beads and grooves, a linker or a linker part may be connected to a capsule compartment, a closure cap, and/or another linker part via a threaded screw- type connection. It is also contemplated that if such a threaded screw-type connection is utilized, a first one of the capsule compartment, closure cap, linker, or linker part may include an external threaded element and a second one of the capsule compartment, closure cap, linker, or linker part may include an internal threaded element configured to be complementary to and engagable with the external threaded element. It is further contemplated that if such a threaded screw-type connection is utilized, the material from which the capsule compartment, closure cap, linker, or linker part may be strengthened via material re-work, additives to the polymer, and/or increased tolerances with respect to the mold or injection process as is known in the art.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A dosage form (100) comprising:
a first generally cylindrical capsule compartment (102) having an axis (104), a closed end (106), and an open end (108);
the open end (108) including an integral, annular linker member (110) extending axially outward of the open end (108) and having an outer surface (120) with a first snap-fit element (122) located a distance L₁ from the capsule compartment open end (108);
a second generally cylindrical capsule compartment (120) having an axis (104), a closed end (123), and an open end (124) with an inner surface (130), the second capsule compartment (120) having a second snap-fit element (128) complementary to the first snap-fit element (122), the second snap-fit element (128) being located on the inner surface (130) and spaced a distance of L₂ from the second capsule open end (124), wherein L₂ > L₁ and the first and second snap-fit elements (122, 128) are engageable to connect the first and second capsule compartments (102, 120) with the respective open ends (108, 124) being in compressive abutting contact with each other.

2. The dosage form (100) of claim 1, wherein the annular linker member (110) is fixed to an inner part (114) of the capsule compartment wall (112) and extending in the axial direction beyond outer wall (116), in a direction away from the closed end (106).

3. The dosage form (100) of claim 2, wherein the radius "r" of the outer surface (120) is less than the radius "R" of the compartment wall (112).

4. The dosage form (100) of any one of the claims 1 to 3, wherein the first snap-fit element is a groove (122) and the complementary second snap-fit element is a ridge (128) configured to engage with the groove (122).

5. The dosage form (100) of any one of the claims 1 to 3, wherein the first snap-fit element is a continuous groove (122) and the second snap-fit element is a continuous ridge (128').

6. The dosage form (100) of any one of the claims 1 to 5, wherein the dosage form (100) is configured as a tablet and has a height (H)/diameter (D) ratio of less than or equal to 1.0.

7. The dosage form (100) of any one of the claims 1 to 6, wherein the walls of compartments (102, 120) further include depressions (134) at the closed compartment ends.
